(19) Europäisches Patentamt
European Patent Office
Office européen des brevets

(11) **EP 3 394 908 B1**

(12) **FASCICULE DE BREVET EUROPEEN**

(45) Date de publication et mention
de la délivrance du brevet:
**12.05.2021 Bulletin 2021/19**

(51) Int Cl.:
***H01L 41/08*** *(2006.01)*    ***H01L 41/312*** *(2013.01)*
***H03H 9/02*** *(2006.01)*

(21) Numéro de dépôt: **16820264.6**

(22) Date de dépôt: **21.12.2016**

(86) Numéro de dépôt international:
**PCT/EP2016/082252**

(87) Numéro de publication internationale:
**WO 2017/109000 (29.06.2017 Gazette 2017/26)**

(54) **SUBSTRAT POUR UN DISPOSITIF A ONDES ACOUSTIQUES DE SURFACE OU A ONDES ACOUSTIQUES DE VOLUME COMPENSE EN TEMPERATURE**

SUBSTRAT FÜR EINE TEMPERATURKOMPENSIERTE OBERFLÄCHENSCHALLWELLENVORRICHTUNG ODER VOLUMENSCHALLWELLENVORRICHTUNG

SUBSTRATE FOR A TEMPERATURE-COMPENSATED SURFACE ACOUSTIC WAVE DEVICE OR VOLUME ACOUSTIC WAVE DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.12.2015 FR 1563058**

(43) Date de publication de la demande:
**31.10.2018 Bulletin 2018/44**

(60) Demande divisionnaire:
**21167505.3**

(73) Titulaire: **Soitec**
**38190 Bernin (FR)**

(72) Inventeurs:
• **BROEKAART, Marcel**
**38570 Theys (FR)**
• **BARGE, Thierry**
**38160 Chevrieres (FR)**
• **GUENARD, Pascal**
**38190 Froges (FR)**
• **RADU, Ionut**
**38920 Crolles (FR)**
• **DESBONNETS, Eric**
**38660 Lumbin (FR)**
• **KONONCHUK, Oleg**
**38570 Theys (FR)**

(74) Mandataire: **Regimbeau**
**20, rue de Chazelles**
**75847 Paris Cedex 17 (FR)**

(56) Documents cités:
**EP-A1- 2 738 939        US-A- 4 019 200**
**US-A1- 2015 102 705**

## Description

## DOMAINE DE L'INVENTION

La présente invention concerne un substrat pour un dispositif à ondes acoustiques de surface ou à ondes acoustiques de volume compensé en température, ainsi qu'un procédé de fabrication d'un tel substrat et un dispositif à ondes acoustiques de surface ou à ondes acoustiques de volume comprenant un tel substrat.

## ARRIERE PLAN DE L'INVENTION

Les dispositifs à ondes acoustiques de surface, généralement désignés par l'acronyme SAW, du terme anglo-saxon « Surface Acoustic Wave », trouvent des applications dans le domaine des communications radio-fréquence (RF), notamment pour des applications à des filtres. Un dispositif SAW comprend typiquement une couche piézoélectrique et deux électrodes sous la forme de deux peignes métalliques interdigités déposés sur la surface de ladite couche piézoélectrique. Un signal électrique, tel qu'une variation de tension électrique, appliqué à une électrode est converti en onde élastique qui se propage à la surface de la couche piézoélectrique. Cette onde est à nouveau convertie en signal électrique en parvenant à l'autre électrode.

Le document EP 2 738 939, le document US 4 019 200, ainsi que le document US 2015/102705 décrivent de tels dispositifs à ondes acoustiques de surface.

Le choix du matériau piézoélectrique tient compte du coefficient du couplage électromécanique qui traduit le taux de la conversion électromécanique par ledit matériau, et de la stabilité en température de la fréquence d'oscillation du matériau piézoélectrique.

Or, les dispositifs SAW sont très sensibles aux variations de température, qui induisent des dilatations différentes de la couche piézoélectrique et des électrodes métalliques dues aux coefficients de dilatation thermique différents de ces matériaux.

Plus précisément, le coefficient de fréquence en température, noté TCF, acronyme du terme anglo-saxon « Temperature Coefficient of Frequency », qui est défini comme la variation de fréquence pour une fréquence donnée f en fonction de la température T, est donné par la formule :

$$TCF = \frac{1}{f}\frac{\partial f}{\partial T} = TCV - CTE$$

où :

$$TCV = \frac{1}{V}\frac{\partial V}{\partial T}$$

V est la vitesse des ondes acoustiques de surface et CTE est le coefficient de dilatation thermique du matériau piézoélectrique dans la direction de propagation des ondes acoustiques de surface.

Il existe déjà des mesures pour compenser les effets de la température sur les dispositifs SAW.

En particulier, l'article de Hashimoto et al [1] présente une revue des différentes techniques de compensation en température des dispositifs SAW.

Parmi ces différentes techniques, on distingue essentiellement :

(1) une technique dite « overlay » consistant à recouvrir la surface de la couche piézoélectrique et les électrodes d'un matériau diélectrique (typiquement, de l'oxyde de silicium ($SiO_2$)), qui présente un coefficient de dilatation thermique de sens opposé à celui de la couche piézoélectrique,

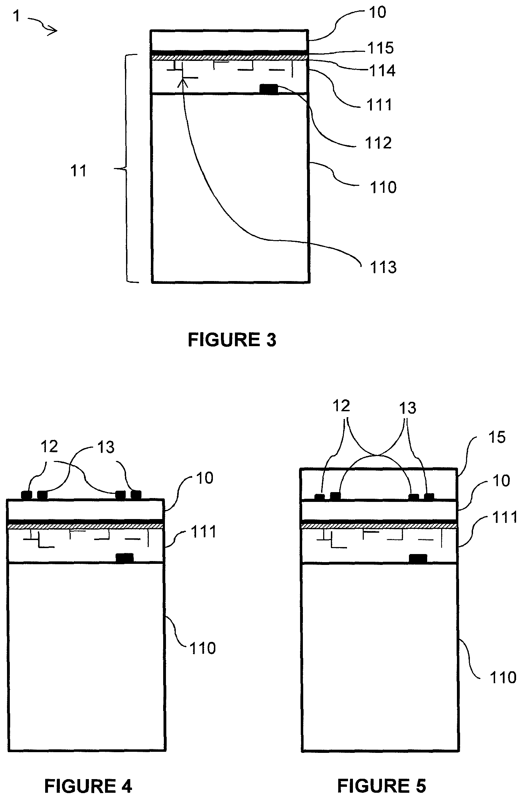

(2) une technique dite « wafer bonding » consistant à coller la couche piézoélectrique sur un substrat support dont le coefficient de dilatation thermique est le plus faible possible de sorte à bloquer la dilatation thermique de la couche piézoélectrique. Ledit substrat support, qui peut être par exemple en silicium, en saphir, en verre ou en spinelle ($MgAl_2O_4$), remplit ainsi une fonction de rigidification de la couche piézoélectrique. Compte tenu de son épaisseur, la couche piézoélectrique est considérée comme s'étendant à l'infini dans une direction opposée aux électrodes, de sorte que la présence du substrat support ne perturbe pas la propagation des ondes acoustiques de surface. Toutefois, le collage du substrat support semble créer des résonances parasites (« spurious résonances ») à des fréquences supérieures à la fréquence principale du dispositif (cf. [1], Fig. 5).

Parmi les matériaux envisagés pour le substrat support dans cette deuxième technique, le silicium semble le plus prometteur car il permet de mettre en oeuvre des procédés d'intégration de composants électroniques à l'échelle du substrat (« wafer level », selon la terminologie anglo-saxonne).

Toutefois, il existe une différence importante de coefficient de dilatation thermique importante entre le matériau piézoélectrique et le silicium (pour un cristal de $LiTaO_3$, qui est anisotrope, les CTE sont de $4\times10^{-6}$/°C et $14\times10^{-6}$/°C environ tandis que le CTE du silicium est de l'ordre de $2,3\times10^{-6}$/°C), qui affecte la stabilité de l'empilement substrat support / couche piézoélectrique si celui-ci est exposé à des températures importantes lors des étapes ultérieures du procédé de fabrication du dispositif à ondes acoustiques de surface. Or, en vue de telles étapes, il est nécessaire d'assurer la stabilité thermique de l'empilement couche piézoélectrique / substrat support jusqu'à une température d'environ 250°C.

Un problème similaire se pose pour les filtres et résonateurs à ondes acoustiques de volume, connus

**EP 3 394 908 B1**

sous l'acronyme BAW (du terme anglo-saxon « Bulk Acoustic Wave »).

Les filtres et résonateurs à ondes acoustiques de volume comprennent typiquement une couche piézoélectrique mince (c'est-à-dire d'épaisseur généralement inférieure à 1 μm) et deux électrodes agencées sur chaque face principale de ladite couche mince. Un signal électrique, tel qu'une variation de tension électrique, appliqué à une électrode est converti en onde élastique qui se propage au travers de la couche piézoélectrique. Cette onde est à nouveau convertie en signal électrique en parvenant à l'électrode située sur la face opposée.

## BREVE DESCRIPTION DE L'INVENTION

Un but de l'invention est de concevoir un substrat pour un dispositif à ondes acoustiques de surface ou pour un dispositif à ondes acoustiques de volume compensé en température qui permette de s'affranchir des inconvénients cités plus haut. En particulier, un tel substrat doit être plus stable que l'empilement substrat support de silicium / couche piézoélectrique mentionné ci-dessus jusqu'à une température d'environ 300°C, tout en permettant une intégration aisée de composants électroniques.

Ce but est atteint par un substrat selon la revendication 1 et un procédé selon la revendication 11. Les réalisations avantageuses sont décrites dans les revendications dépendantes.

## BREVE DESCRIPTION DES DESSINS

D'autres caractéristiques et avantages de l'invention ressortiront de la description détaillée qui va suivre, en référence aux dessins annexés sur lesquels :

- la figure 1 est une vue de principe en coupe d'un filtre à ondes acoustiques de surface compensé en température,
- la figure 2 est une vue de principe en coupe d'un résonateur à ondes acoustiques de volume compensé en température,
- la figure 3 est un schéma en coupe d'un substrat selon une forme d'exécution de l'invention,
- la figure 4 est un schéma en coupe d'un filtre à ondes acoustiques de surface compensé en température selon un mode de réalisation de l'invention,
- la figure 5 est un schéma en coupe d'un filtre à ondes acoustiques de surface compensé en tmpérature selon une variante de réalisation de l'invention,
- les figures 6A à 6E illustrent des étapes successives de la fabrication d'un substrat selon un mode de réalisation de l'invention.

Pour des raisons de lisibilité des figures, les éléments illustrés ne sont pas nécessairement représentés à l'échelle. Par ailleurs, les éléments désignés par les mêmes signes de référence sur différentes figures sont identiques.

## DESCRIPTION DETAILLEE DE MODES DE REALISATION DE L'INVENTION

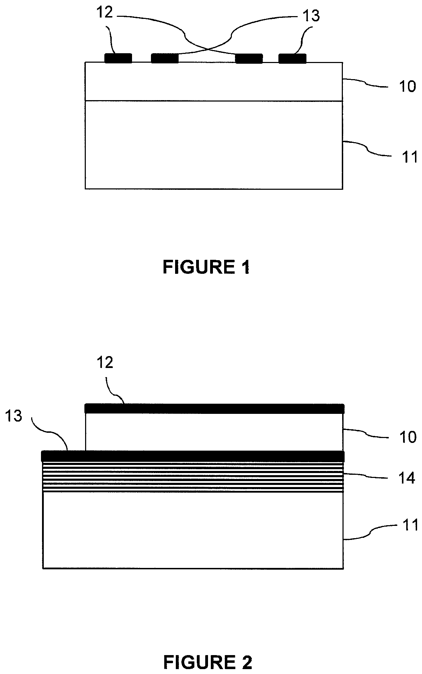

La figure 1 est une vue de principe d'un filtre à ondes acoustiques de surface.

Ledit filtre comprend une couche piézoélectrique 10 et deux électrodes 12, 13 sous la forme de deux peignes métalliques interdigités déposés sur la surface de ladite couche piézoélectrique. Du côté opposé aux électrodes 12, 13, la couche piézoélectrique repose sur un substrat support 11 destiné à assurer une compensation en température, et dont la structure sera décrite en détail plus bas. La couche piézoélectrique 10 présente de préférence une excellente qualité cristalline pour ne pas engendrer d'atténuation de l'onde de surface. Ladite couche est donc monocristalline. A l'heure actuelle, les matériaux adéquats utilisables industriellement sont le quartz, le $LiNbO_3$ ou le $LiTaO_3$. La couche piézoélectrique 10 est généralement obtenue par découpe d'un lingot de l'un desdits matériaux, la précision requise pour l'épaisseur de ladite couche étant peu importante dans la mesure où les ondes doivent se propager essentiellement à sa surface.

La figure 2 est une vue de principe d'un résonateur à ondes acoustiques de volume.

Le résonateur comprend une couche piézoélectrique mince (c'est-à-dire d'épaisseur généralement inférieure à 20 nm) et deux électrodes 12, 13 agencées de part et d'autre de ladite couche piézoélectrique 10. La couche piézoélectrique 10 repose sur un substrat support 11 dont la structure sera décrite en détail plus bas. Pour isoler le résonateur du substrat et éviter ainsi la propagation des ondes dans le substrat, un miroir de Bragg 14 est interposé entre l'électrode 13 et le substrat 11. De manière alternative (non illustré), cette isolation pourrait être réalisée en ménageant une cavité entre le substrat et la couche piézoélectrique. Ces différentes dispositions sont connues de l'homme du métier et ne seront donc pas décrites en détail dans le présent texte.

Pour un dispositif à ondes acoustiques de volume, la couche piézoélectrique 10 présente une épaisseur déterminée et uniforme sur l'ensemble de la couche. En revanche, la qualité cristalline ne revêtant pas une importance particulière pour les performances du résonateur, un matériau piézoélectrique polycristallin est acceptable. La couche piézoélectrique 10 est donc généralement formée par dépôt sur un support (par exemple un substrat de silicium). A l'heure actuelle, les matériaux employés industriellement pour un tel dépôt sont l'AIN, le ZnO et le PZT.

La figure 3 est un schéma en coupe d'un substrat permettant de fabriquer un dispositif à ondes acoustiques de surface ou à ondes acoustiques de volume compensé en température selon une forme d'exécution de l'invention.

Ledit substrat 1 comprend une couche piézoélectrique 10 destinée à recevoir des électrodes pour former un dispositif à ondes acoustiques de surface ou de volume.

Le matériau, la qualité cristalline et l'épaisseur de ladite couche piézoélectrique 10 sont choisis par l'homme du métier en fonction de l'application visée. Les critères de choix sont connus en eux-mêmes et ne nécessitent pas d'être décrits en détail dans le présent texte.

Si le matériau piézoélectrique choisi est anisotrope, celui-ci présente des coefficients de dilatation thermique différents dans des directions différentes.

La couche piézoélectrique 10 est disposée sur un substrat support 11 composite, c'est-à-dire formé d'un empilement de plusieurs couches de matériaux différents.

Ledit substrat support 11 comprend un substrat 110 dit raidisseur dont la fonction, au sein du substrat 1, est d'assurer la rigidité de l'empilement notamment lors de traitements thermiques.

Le substrat raidisseur 110 comprend avantageusement du saphir, du verre et/ou de la spinelle ($MgAl_2O_4$).

Ces matériaux présentent l'avantage de présenter un coefficient de dilatation thermique plus proche du coefficient de dilatation thermique du matériau piézoélectrique que le silicium, ce qui permet une meilleure stabilité en température (jusqu'à 300°C environ) de l'empilement, bien que cette plus grande proximité des coefficients de dilatation thermique pénalise légèrement l'effet de compensation en température.

Le substrat raidisseur 110 présente par ailleurs une épaisseur élevée, typiquement de l'ordre de 400 à 800 $\mu$m, qui est bien supérieure à l'épaisseur des autres couches du substrat 1 et notamment bien supérieure à l'épaisseur de la couche piézoélectrique, laquelle est généralement inférieure à 50 $\mu$m, de préférence inférieure à 20 $\mu$m, et de manière encore préférée inférieure à 1 $\mu$m. Ainsi, le comportement en température du substrat raidisseur est prépondérant par rapport à celui des autres couches.

Du fait de la relative proximité entre le coefficient de dilatation thermique du substrat raidisseur 110 et de la couche piézoélectrique 10, les contraintes dues à la différence de coefficients de dilatation thermique lors de traitements thermiques subis par le substrat 1 sont minimisées.

Entre le substrat raidisseur 110 et la couche piézoélectrique 10 est intercalée une couche semi-conductrice 111. Ladite couche semi-conductrice peut comprendre du silicium, du germanium, du SiGe, du SiC, ou encore un matériau de type III-V, tel que GaAs, GaN, InGaN (liste non limitative). Parmi ces matériaux, le germanium et le GaAs sont moins préférés en raison de leur fragilité. Selon une forme d'exécution préférée de l'invention, la couche semi-conductrice est une couche de silicium.

De manière particulièrement avantageuse, la couche semi-conductrice 111 comprend au moins un composant électronique 112. Ledit composant est fabriqué par des techniques connues dans la microélectronique. Il peut ainsi s'agir d'un transistor CMOS, d'un commutateur (switch), d'un amplificateur de puissance (liste non limitative). Par ailleurs, des vias 113 peuvent être ménagés à l'intérieur de la couche semi-conductrice 111 de manière à permettre à connecter électriquement différents composants. Ces composants et vias sont formés par des techniques conventionnelles en microélectronique qui ne seront pas décrites en détail dans le présent texte.

La couche semi-conductrice 111 est sensiblement plus fine que le substrat raidisseur 110. Ainsi, la couche semi-conductrice 111 présente typiquement une épaisseur comprise entre 10 nm et 2 $\mu$m. Par conséquent, même si le matériau de la couche semi-conductrice présente une différence de coefficient de dilatation thermique vis-à-vis du matériau piézoélectrique plus grande que la différence de coefficient de dilatation thermique entre le matériau du substrat raidisseur 110 et le matériau piézoélectrique 10, la couche semi-conductrice 111 est suffisamment fine pour ne pas générer de contrainte mécanique dans la couche piézoélectrique 10 lors d'un traitement thermique.

En outre, par rapport à un substrat massif de saphir, le substrat composite 11 formé du substrat raidisseur 110 de saphir et de la couche semi-conductrice 111 permet l'intégration de composants électroniques en face arrière de la couche piézoélectrique 10.

Selon l'invention, une couche diélectrique 114 est agencée à l'interface entre la couche semi-conductrice 111 et la couche piézoélectrique 10. Une telle couche diélectrique est généralement utilisée pour favoriser le collage de la couche piézoélectrique 10 sur la couche semi-conductrice 111. La couche diélectrique peut être formée, préalablement au collage de la couche piézoélectrique 10 sur la couche semi-conductrice 111, soit sur l'une seule de ces couches, soit sur chacune d'elles (un collage de type oxyde sur oxyde étant réalisé dans ce dernier cas). Une couche de piégeage de charges 115 est réalisée sous la couche piézoélectrique, avantageusement interposée entre la couche diélectrique 14 et la couche piézoélectrique 10 ou entre la couche semi-conductrice 111 et la couche diélectrique 114 afin de piéger les charges électriques présentes qui seraient susceptibles de perturber le fonctionnement des composants électroniques agencés dans la couche semi-conductrice 111. Ladite couche 115 de piégeage peut comprendre par exemple une couche de silicium polycristallin ou amorphe. Toutefois, toute autre couche (ou empilement de couches) remplissant la fonction de piégeage de charges électriques peut être employée.

La figure 4 illustre de manière schématique un filtre à ondes acoustiques de surface formé sur le substrat 1 de la figure 3. A cet effet, des électrodes métalliques 12, 13 ont été déposées sous la forme de deux peignes interdigités sur la surface libre de la couche piézoélectrique 10.

Selon une variante d'exécution illustrée à la figure 5, le substrat peut, après formation des électrodes 12, 13 sur la couche piézoélectrique 10, être recouvert d'une couche 15 de diélectrique (typiquement, de l'oxyde de silicium ($SiO_2$)), selon la technique d'« overlay » mentionnée plus haut. L'épaisseur de la couche diélectrique 15 est typiquement de l'ordre de 100 à 10000 nm.

Par rapport au mode de réalisation de la figure 4, la couche diélectrique 15, qui présente un coefficient de dilatation thermique de sens opposé à celui de la couche piézoélectrique 10, permet d'améliorer la compensation en température.

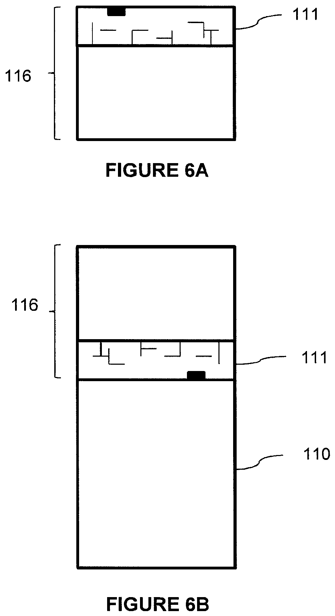

On va maintenant décrire, en référence aux figures 6A à 6E, un procédé de fabrication d'un substrat pour un dispositif à ondes acoustiques de surface ou à ondes acoustiques de volume selon un mode de réalisation non limitatif de l'invention. Le procédé décrit ci-dessous implique un collage puis un amincissement d'un substrat donneur, mais d'autres techniques, telles qu'une approche de type « substrat démontable » comme décrit dans le document FR 2 816 445, pourraient être employées. Un tel substrat « démontable » est réalisé avant la fabrication des composants et contient une zone ou interface de fragilisation permettant la fracture du substrat donneur après l'assemblage sur un substrat raidisseur.

En référence à la figure 6A, on fournit un substrat donneur 116 comprenant la couche semi-conductrice 111 dans laquelle sont avantageusement intégrés des composants électroniques 112 et/ou des vias 113, selon des techniques couramment utilisées en microélectronique.

En référence à la figure 6B, on colle le substrat donneur 116 sur le substrat raidisseur 110, de sorte que la couche semi-conductrice 111 se trouve à l'interface de collage.

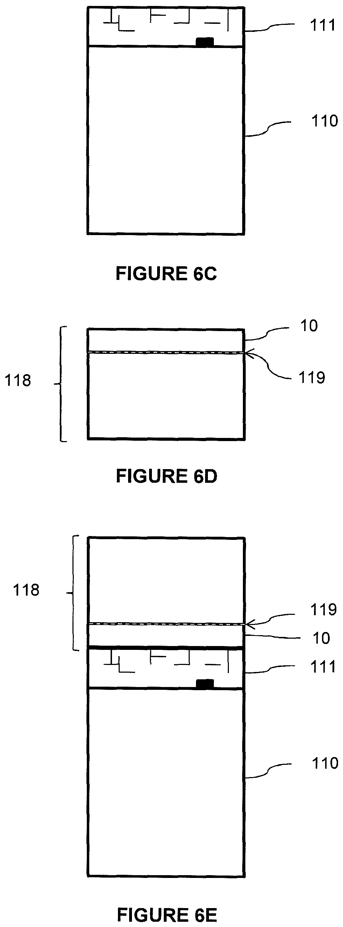

En référence à la figure 6C, on amincit le substrat donneur par la face opposée à la couche semi-conductrice 111 de sorte à transférer la couche comprenant la couche 111 sur le substrat raidisseur 110. Ledit amincissement peut être mécanique (de type polissage), chimique (gravure), ou autre. Le cas échéant, on intègre ensuite à ladite couche des composants électroniques et/ou des vias.

En référence à la figure 6D, on fournit un substrat donneur 118 d'un matériau piézoélectrique et l'on forme par implantation d'espèces atomiques dans ledit substrat une zone de fragilisation 119 délimitant une couche piézoélectrique à transférer, à savoir la couche piézoélectrique 10 du substrat final illustré sur la figure 3. Les conditions d'implantation sont connues dans l'état de la technique, à savoir une dose de l'ordre de 5 à 15E16 et une énergie comprise entre 20 et 200 keV.

En référence à la figure 6E, on colle le substrat donneur 118 sur l'empilement formé du substrat raidisseur 110 et de la couche semi-conductrice 111, de sorte que la couche semi-conductrice 111 et la couche piézoélectrique 10 soient à l'interface de collage. Comme mentionné plus haut, une couche diélectrique (non illustrée sur la figure 6E) est formée au préalable sur l'une et/ou l'autre de ces couches afin de favoriser le collage. Une couche de piégeage de charges (non illustrée sur la figure 6E) peut être formée entre ladite couche diélectrique et la couche piézoélectrique. Ladite couche de piégeage est avantageusement formée après l'implantation réalisée dans le substrat piézoélectrique 118. Dans ces conditions, un procédé à basse température est requis. Par exemple, une couche de silicium amorphe est déposée sur le substrat piézoélectrique, ou une couche de silicium polycristallin est déposée sur une couche diélectrique formée sur la couche semi-conductrice 111.

Dans le cas où l'on souhaite former un dispositif à ondes acoustiques de volume, le collage peut être réalisé par l'intermédiaire d'une couche métallique, ladite couche remplissant alors la fonction d'électrode enterrée dans le dispositif.

Ensuite, on fracture le substrat donneur piézoélectrique 118 le long de la zone de fragilisation 119 de sorte à transférer la couche piézoélectrique 10 sur la couche semi-conductrice 111. Un amincissement de la couche piézoélectrique peut éventuellement être mis en oeuvre pour éliminer des défauts liés à l'implantation.

Dans le cas où la couche semi-conductrice ne comprend pas de composants électroniques, le procédé Smart Cut™ peut également être mis en oeuvre pour transférer la couche semi-conductrice 111 sur le substrat raidisseur 110. Ce procédé est bien connu de l'homme du métier. En particulier, on forme par implantation d'espèces atomiques une zone de fragilisation dans le substrat donneur 116, de sorte à délimiter une couche à transférer comprenant la couche 111. Cette implantation met généralement en oeuvre des atomes d'hydrogène et/ou d'hélium, l'homme du métier étant à même de déterminer la dose et l'énergie d'implantation en fonction du matériau du substrat donneur et de la profondeur à atteindre. Puis, après le collage du substrat donneur sur le substrat raidisseur 110, on détache le substrat donneur le long de la zone de fragilisation, ce détachement pouvant être initié de manière mécanique, thermique, chimique ou autre.

Dans le cas où l'on souhaite fabriquer un dispositif à ondes acoustiques de surface, on dépose ensuite, sur la surface de la couche piézoélectrique 10, des électrodes métalliques sous la forme de deux peignes interdigités.

Dans le cas où l'on souhaite fabriquer un dispositif à ondes acoustiques de volume, une adaptation des étapes décrites ci-dessus doit être effectuée. D'une part, on dépose, avant l'étape de collage illustrée sur la figure 6E, une première électrode sur la surface libre de la couche piézoélectrique 10 faisant partie du substrat donneur piézoélectrique, cette première électrode se trouvant enterrée dans l'empilement final. Après le transfert de la couche piézoélectrique 10 sur la couche semi-conductrice 111, on dépose une seconde électrode sur la surface libre de la couche piézoélectrique, opposée à la première électrode. D'autre part, pour éviter la propagation des ondes acoustiques dans la couche semi-conductrice 111 et dans le substrat raidisseur 110, on intègre à la couche semi-conductrice 111 un moyen d'isolation pouvant être, par exemple, un miroir de Bragg (comme illustré sur la figure 2) ou une cavité gravée dans la couche semi-conductrice 111.

**REFERENCES**

[1] Hashimoto et al, Recent Development of Temperature Compensated SAW Devices, Ultrasonics Symposium (IUS), 18-21 Oct. 2011, pp. 79-86, 2011 IEEE International

**Revendications**

1. Substrat (1) pour un dispositif à ondes acoustiques de surface ou à ondes acoustiques de volume, comprenant un substrat support (11) et une couche piézoélectrique (10) sur ledit substrat support, **caractérisé en ce que** le substrat support (11) comprend une couche semi-conductrice (111) sur un substrat raidisseur (110) présentant un coefficient de dilatation thermique plus proche du coefficient de dilatation thermique du matériau de de la couche piézoélectrique (10) que celui du silicium, la couche semi-conductrice (111) étant agencée entre la couche piézoélectrique (10) et le substrat raidisseur (110), le substrat (1) étant **caractérisé en ce qu'**il comprend en outre une couche diélectrique (114) entre la couche piézoélectrique (10) et la couche semi-conductrice (111) et une couche (115) de piégeage de charges à l'interface entre ladite couche diélectrique et la couche semi-conductrice et/ou l'interface entre la couche diélectrique et la couche piézoélectrique.

2. Substrat selon la revendication 1, **caractérisé en ce que** le substrat raidisseur (110) comprend du saphir, du verre et/ou de la spinelle ($MgAl_2O_4$).

3. Substrat selon l'une des revendications 1 ou 2, **caractérisé en ce que** la couche semi-conductrice (111) est formée de l'un des matériaux suivants: silicium, germanium, SiGe, SiC, matériau III-V.

4. Substrat selon l'une des revendications 1 à 3, **caractérisé en ce que** la couche semi-conductrice (111) comprend au moins un composant électronique (112).

5. Substrat selon la revendication 4, **caractérisé en ce que** ledit composant électronique (112) est choisi parmi : un transistor CMOS, un commutateur et un amplificateur de puissance.

6. Substrat selon l'une des revendications 1 à 5, **caractérisé en ce que** le ratio entre l'épaisseur de la couche piézoélectrique (10) et l'épaisseur du substrat raidisseur (110) est inférieur ou égal à 0,125.

7. Substrat selon l'une des revendications 1 à 6, **caractérisé en ce que** l'épaisseur de la couche piézoélectrique (10) est inférieure à 50 µm, de préférence inférieure à 20 µm, de manière encore préférée inférieure à 1 µm, et l'épaisseur du substrat raidisseur (110) est comprise entre 400 et 800 µm.

8. Substrat selon l'une des revendications 1 à 7, **caractérisé en ce que** ladite couche (115) de piégeage de charges comprend une couche de silicium polycristallin.

9. Dispositif à ondes acoustiques de surface comprenant un substrat (1) selon l'une des revendications 1 à 8 et deux électrodes (12, 13) formées de deux peignes métalliques interdigités sur la surface de la couche piézoélectrique (10).

10. Dispositif à ondes acoustiques de volume comprenant un substrat (1) selon l'une des revendications 1 à 8 et deux électrodes (12, 13) agencées de part et d'autre de la couche piézoélectrique (10).

11. Procédé de fabrication d'un substrat (1) selon l'une des revendications 1 à 8, **caractérisé en ce qu'**il comprend :

    - le transfert de la couche semi-conductrice (111) sur le substrat raidisseur (110) à partir d'un premier substrat donneur (116),
    - le transfert de la couche piézoélectrique sur la couche semi-conductrice (111) à partir d'un second substrat donneur (118).

12. Procédé selon la revendication 11, dans lequel l'une au moins des étapes de transfert comprend les sous-étapes suivantes :

    - formation d'une zone de fragilisation dans le premier, respectivement le second substrat donneur par implantation d'espèces atomiques ;
    - collage du premier, respectivement du second substrat donneur sur le substrat raidisseur, respectivement sur la couche semi-conductrice ;
    - détachement dudit premier, respectivement second, substrat le long de la zone de fragilisation.

**Patentansprüche**

1. Substrat (1) für eine Vorrichtung mit akustischen Oberflächenwellen oder mit akustischen Volumenwellen, umfassend ein Trägersubstrat (11) und eine piezoelektrische Schicht (10) auf dem genannten Trägersubstrat, **dadurch gekennzeichnet, dass** das Trägersubstrat (11) eine Halbleiterschicht (111) auf einem Versteifungssubstrat (110) umfasst, das einen thermischen Ausdehnungskoeffizienten aufweist, der näher an dem thermischen Ausdehnungskoeffizienten des Materials der piezoelektrischen Schicht (10) ist als der des Siliziums, wobei die Halbleiterschicht (111) zwischen der piezoelektrischen Schicht (10) und dem Versteifungssubstrat (110) angeordnet ist, wobei das Substrat (1) **dadurch gekennzeichnet ist, dass** es darüber hinaus eine dielektrische Schicht (114) zwischen der piezoelektrischen Schicht (10) und der Halbleiterschicht (111) und eine Chargen-Einfangschicht (115) an der Schnittstelle zwischen der genannten dielektrischen Schicht und der Halbleiterschicht und/oder der Schnittstelle zwischen der dielektrischen Schicht und der piezoelektrischen Schicht umfasst.

2. Substrat gemäß Anspruch 1, **dadurch gekennzeichnet, dass** das Versteifungssubstrat (110) Saphir, Glas und/oder Spinell ($MgAl_2O_4$) umfasst.

3. Substrat gemäß einem der Ansprüche 1 oder 2, **dadurch gekennzeichnet, dass** die Halbleiterschicht (111) aus einem der folgenden Materialien gebildet ist: Silizium, Germanium, SiGe, SiC, Material III-V.

4. Substrat gemäß einem der Ansprüche 1 bis 3, **dadurch gekennzeichnet, dass** die Halbleiterschicht (111) wenigstens ein elektronisches Bauteil (112) umfasst.

5. Substrat gemäß Anspruch 4, **dadurch gekennzeichnet, dass** das genannte elektronische Bauteil (112) ausgewählt ist aus: einem Transistor CMOS, einem Umschalter und einem Leistungsverstärker.

6. Substrat gemäß einem der Ansprüche 1 bis 5, **dadurch gekennzeichnet, dass** das Verhältnis zwischen der Dicke der piezoelektrischen Schicht (10) und der Dicke des Versteifungssubstrats (110) geringer als oder gleich 0,125 ist.

7. Substrat gemäß einem der Ansprüche 1 bis 6, **dadurch gekennzeichnet, dass** die Dicke der piezoelektrischen Schicht (10) geringer ist als 50 $\mu$m, bevorzugt geringer al 20 $\mu$m, weiter bevorzugt geringer als 1 $\mu$m, und die Dicke des Versteifungssubstrats (110) zwischen 400 und 800 $\mu$m inbegriffen ist.

8. Substrat gemäß einem der Ansprüche 1 bis 7, **dadurch gekennzeichnet, dass** die genannte Chargen-Einfangschicht (115) eine polykristalline Siliziumschicht umfasst.

9. Vorrichtung mit akustischen Oberflächenwellen, umfassend ein Substrat (1) gemäß einem der Ansprüche 1 bis 8 und zwei Elektroden (12, 13), die aus zwei interdigitalen Metallkämmen auf der Oberfläche der piezoelektrischen Schicht (10) gebildet sind.

10. Vorrichtung mit akustischen Volumenwellen, umfassend ein Substrat (1) gemäß einem der Ansprüche 1 bis 8 und zwei Elektroden (12, 13), die auf jeder Seite der piezoelektrischen Schicht (10) angeordnet sind.

11. Herstellungsverfahren eines Substrats (1) gemäß einem der Ansprüche 1 bis 8, **dadurch gekennzeichnet, dass** es umfasst:

    - die Übertragung der Halbleiterschicht (111) auf das Versteifungssubstrat (110) ausgehend von einem ersten Spendersubstrat (116),
    - die Übertragung der piezoelektrischen Schicht auf die Halbleiterschicht (111) ausgehend von einem zweiten Spendersubstrat (118).

12. Verfahren gemäß Anspruch 11, bei dem wenigstens einer der Übertragungsschritte die folgenden Teilschritte umfasst:

    - Bildung eines Bruchbereichs in dem ersten beziehungsweise dem zweiten Spendersubstrat durch Einsetzen von Atomsorten;
    - Verkleben des ersten beziehungsweise des zweiten Spendersubstrats auf dem Versteifungssubstrat beziehungsweise auf der Halbleiterschicht;
    - Ablösen des ersten beziehungsweise des zweiten Substrats entlang des Bruchbereichs.

**Claims**

1. Substrate (1) for a surface acoustic wave device or bulk acoustic wave device which comprises a support substrate (11) and an piezoelectric layer (10) on said support substrate, **characterised in that** the support substrate (11) comprises a semiconductor layer (111) on a stiffening substrate (110) which has a coefficient of thermal expansion closer to the coefficient of thermal expansion of the material of the piezoelectric layer (10) than that of silicon, where the semiconductor layer (111) is arranged between the piezoelectric layer (10) and the stiffening substrate (110), the substrate (1) being **characterised in that** it further comprises a dielectric layer (114) between the piezoelectric layer (10) and the semiconductor layer (111) and a charge trapping layer (115) at the interface between said dielectric layer and the semiconductor layer and/or the interface between the dielectric layer and the piezoelectric layer.

2. Substrate according to claim 1, **characterised in that** the stiffening substrate (110) comprises sapphire, glass and/or spinel (MgAl$_2$O$_4$).

3. Substrate according to one of claims 1 or 2 **characterised in that** the semiconductor layer (111) is formed from one of the following materials: silicon, germanium, SiGe, SiC, III-V material.

4. Substrate according to one of claims 1 to 3 **characterised in that** the semiconductor layer (111) comprises at least one electronic component (112).

5. Substrate according to claim 4, **characterised in that** said electronic component (112) is chosen from: a CMOS transistor, a switch and a power amplifier.

6. Substrate according to one of claims 1 to 5, **characterised in that** the ratio of the thickness of the piezoelectric layer (10) to the thickness of the stiffening substrate (110) is less than or equal to 0.125.

7. Substrate according to one of claims 1 to 6, **characterised in that** the thickness of the piezoelectric layer (10) is less than 50 $\mu$m, preferably less than 20 $\mu$m, yet more preferably less than 1 $\mu$m, and the thickness of the stiffening substrate (110) is between 400 and 800 $\mu$m.

8. Substrate according to one of claims 1 to 7, **characterised in that** said charge trapping layer (115) comprises a layer of polycrystalline silicon.

9. Surface acoustic wave device comprising a substrate (1) according to one of claims 1 to 8 and two electrodes (12, 13) formed of two interdigitated metallic combs on the surface of the piezoelectric layer (10).

10. Bulk acoustic wave device comprising a substrate (1) according to one of claims 1 to 8 and two electrodes (12, 13) arranged on either side of the piezoelectric layer (10).

11. Method of manufacturing a substrate (1) according to one of claims 1 to 8, **characterised in that** it comprises:

   - the transfer of the semiconductor layer (111) onto the stiffening substrate (110) from a first donor substrate (116),
   - the transfer of the piezoelectric layer onto the semiconductor layer (111) from a second donor substrate (118).

12. Method according to claim 11, wherein at least one of the transfer steps comprises the following substeps:

   - formation of an embrittlement zone in the first, respectively second, donor substrate by implantation of atomic species;
   - bonding of the first, respectively second, donor substrate onto the stiffening substrate or semiconductor layer, respectively;
   - detachment of said first, respectively second, substrate along the embrittlement zone.

12         13

10

11

**FIGURE 1**

12

13

10

14

11

**FIGURE 2**

**FIGURE 3**

**FIGURE 4**

**FIGURE 5**

**FIGURE 6A**

**FIGURE 6B**

111

110

**FIGURE 6C**

10

118

119

**FIGURE 6D**

118

119

10

111

110

**FIGURE 6E**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2738939 A
- US 4019200 A
- US 2015102705 A
- FR 2816445

**Littérature non-brevet citée dans la description**

- Recent Development of Temperature Compensated SAW Devices. **HASHIMOTO et al.** Ultrasonics Symposium (IUS). IEEE, 18 Octobre 2011, 79-86